# EUROPEAN PATENT SPECIFICATION

(11) **EP 4 358 843 B1**
(45) Date of publication and mention of the grant of the patent: **02.07.2025**
(21) Application number: 21736989.1
(22) Date of filing: 22.06.2021
(51) Int. Cl.: A61B 5/256, A61B 5/291, A61B 5/31, A61B 5/00

(54) **SIZE-ADJUSTABLE EEG HEADSET**
EEG-HEADSET MIT EINSTELLBARER GRÖSSE
CASQUE D'ÉLECTROENCÉPHALOGRAMME À TAILLE RÉGLABLE

(43) Date of publication of application: 01.05.2024
(73) Proprietor: Cumulus Neuroscience Limited, Belfast BT3 9DT (GB)
(72) Inventor: MCCULLOUGH, Ian, Comber, Down BT23 5FW (GB); MURPHY, Bryan, Dublin, K78 VH98 (IE); GRIFFITHS, Glyn, Dublin, D14 K284 (IE); KEALY, Terence, Kilkenny, R95YF9H (IE); URASINI, Sara, Corke Abbey A98 CE55 Bray, Co. Wicklow (IE)
(74) Representative: Heeschen Pültz Patentanwälte PartGmbB
(86) International application number: PCT/EP2021/066991
(87) International publication number: WO 2022/268299

(56) References cited:
- CN-U- 204 207 746
- CN-U- 211 513 049
- US-A1- 2005 197 556
- US-A1- 2011 098 593
- US-A1- 2018 132 746

## Description

### Technical Field

The present disclosure generally relates to a size-adjustable headset and an electroencephalography monitoring system.

### Background

Measurements of bioelectrical signals such as electroencephalography, EEG, or electrocardiography, ECG, measurements are usually obtained via electrodes placed on a surface of a subject's (e.g., an animal's or a human's) body. For instance, EEG caps comprising a plurality of so-called wet electrodes are known in the art. Such electrodes are usually permanently fixed to the EEG cap and can obtain EEG measurements when contacting the wearer's head surface via a conductive gel.

Common EEG caps are designed to fit a certain head size only (e.g., a head circumference of 60 cm). Additionally, heads not only differ from one another in size, but also in shape. This may lead to a loose fit of common EEG caps, possibly resulting in an inaccurate electrode placement on the scalp, or to an excessively tight fit potentially resulting in discomfort of the wearer.

If EEG caps are to be used outside a clinical setup, for example at home, additional problems may arise. For instance, such EEG caps should be portable, easy to use, sturdy, and comfortable for the wearer. Furthermore, a reliant electrode placement should be provided by ensuring a good fit of the EEG cap for the user of interest. Additionally, several parts may need replacement from time to time.

Prior art is known from US 2011/098593 A1, CN 211 513 0469 U, US 2005/197556 A1, US 2018/132746 A1 und CN 204 207 746 U.

### Summary

There is a need for a headset that solves one or more of the aforementioned or other problems.

The invention is defined by the independent claim. Specific embodiments are defined by the dependent claims.

According to a first aspect, a size-adjustable headset for monitoring bioelectrical signals is provided. The headset comprises a headband. The headband may be configured to be worn by a user. The headband may be configured to be put on or around a user's head. The headband comprises a first connector element, at least one side band comprising a second connector element distant from a first end of the at least one side band, and at least one electrode contact configured to be electrically connected to a measurement electrode. The first connector element and the second connector element are configured to be (e.g., removably) connected to one another such that a length of the at least one side band between the first end and the first connector element can be adjusted.

The bioelectrical signals may comprise electroencephalographic, EEG, signals. The headset may be an EEG headset. The measurement electrode may be an EEG electrode, for example a dry electrode or a wet electrode. Preferred is the use of a dry electrode. The headband may be configured to encircle at least a portion of the user's head when put on the user's head. The headband may have an inner circumference matching a circumference of a human head.

The first end may be a first longitudinal end of the at least one side band. The second connector element may be arranged at or attached to a second longitudinal end of the at least one side band. The first longitudinal end may be opposite to the second longitudinal end. The first connector element and the second connector element may be configured to couple to one another.

The at least one electrode contact may be configured to be physically (e.g., directly) connected to the measurement electrode. The at least one electrode contact may be configured to contact the measurement electrode via a pressure contact or a plug contact. One or more of the at least one electrode contact may be arranged at (e.g., arranged on, embedded in or attached to) the headband. Alternatively or additionally, one or more of the at least one electrode contact may be arranged at (e.g., arranged on, embedded in or attached to) the at least one side band.

The first connector element and the second connector element may be configured to be connected to one another such that the length of the at least one side band between the first end and the first connector element can be adjusted between a plurality (e.g., 3 or more) of predefined lengths. The first connector element and the second connector element may be configured to be connected to one another such that the distance between the first end and the first connector element can be adjusted between a plurality (e.g., 3 or more) of predefined distances. In the following, while reference will be made to specific lengths, the same applies to distances between the first end and the first connector element.

The plurality of predefined lengths may be equally distributed in a range of lengths. The plurality of predefined lengths my comprise or consist of lengths that differ from one another by a similar or equal amount (e.g., an amount between 0.3 cm and 1.5 cm, for example 0.6 cm). The length between the first end and the first connector element may be adjusted with increments, such as 0.6 cm. For instance, the length may be adjustable between 12 cm, 12.6 cm, 13.2 cm and 13.8 cm. The skilled person will know how to adjust the base length and the predefined lengths depending on the size of a specific user's head. The plurality of predefined lengths may comprise a longest length and a shortest length deviating from one another by a predefined offset (e.g., an offset between 0.5 cm and 5 cm, for example 1.2 cm). The predefined offset may be a multiple of the similar amount.

In one embodiment, the first connector element and the second connector element are configured to be connected to one another in a plurality of predefined positions. These predefined positions may be relative to another.

The headset further comprises a protection unit configured to, when the first connector element and the second connector element are connected to one another and when the protection unit is in a first state, block a disconnection of the first connector element and the second connector element. The protection unit is configured to, when the first connector element and the second connector element are connected to one another and when the protection unit is in a second state, allow the disconnection of the first connector element and the second connector element. The protection unit may comprise a cap or cover configured to be put on the first connector element and the second connector element.

The protection unit comprises a key mechanism configured to, when actuated by a key, shift the protection unit from the first state to the second state and/or from the second state to the first state. In an alternative, the key mechanism may only be configured to, when actuated by a key, shift the protection unit from one state to the other, while not requiring any actuation by a key in the reverse direction. For instance, the key mechanism may be used to shift the protection unit from the first state to the second state and allow disconnection of the first connector element and the second connector element, while the protection unit may automatically, without requiring any actuation by a key mechanism, shift from the second state to the first state to block disconnection, once the protection unit is put on the first connector element and the second connector element.

At least one of the first connector element and the second connector element may comprise a plurality of fixture elements spaced apart from one another (e.g., by the similar or equal amount defined above) in a (e.g., longitudinal) direction of the at least one side band (e.g., when the first connector element is connected to the second connector element). The respective other of the first connector element and the second connector element may comprise at least one fixture counterpart configured to couple to one or more of the plurality of fixture elements.

The plurality of fixture elements may comprise at least one of a male or female coupling element. The at least one fixture counterpart may comprise the respective other of the male or female coupling element. The female coupling element may comprise at least one of a notch, a catch and a hole. The male coupling element may comprise at least one of a pin, a stud and a bolt. In one example, the plurality of fixture elements comprises a (e.g., at least one) hole and the at least one fixture counterpart comprises a pin configured to at least partially extend into the (e.g., at least one) hole. The plurality of fixture elements may comprise a plurality of holes. The pin may be configured to at least partially extend into one of the plurality of holes at a time.

The (e.g., at least one) hole is for example a through-hole, wherein the pin is configured to extend into the hole from a first side of the hole, and wherein the pin is configured to protrude from the hole from a second side of the hole, wherein the second side is opposite to the first side. The pin may be configured to completely extend throughout the hole.

The hole may extend in a lateral direction of the at least one sideband and the pin may extend in a lateral direction of the headband. Alternatively or additionally, the pin may extend in a lateral direction of the at least one sideband and the hole may extend in a lateral direction of the headband. The lateral direction may be the anatomical lateral direction of a user when wearing the headset. The lateral direction may extend generally perpendicular to an outer surface of the headband and/or the at least one sideband. The lateral direction may extend generally perpendicular to a surface of a head of a user when the user is wearing the headset.

The pin may comprise a first axial end and a second axial end, the first axial end being closer to the headband than the second axial end, wherein the pin comprises an end element at the second axial end, the end element being radially wider than a part of the pin adjacent to the end element. The pin may be generally cylindrical.

The pin may have a circumferential notch adjacent to the end element. The pin may have a widened pin head as the end element.

The protection unit may be configured to, in the first state, couple to the end element of the pin to restrict movement of the hole and/or the side band relative to the pin in (e.g., at least) an axial (e.g., longitudinal) direction of the pin. The axial direction of the pin may be parallel or equal to the lateral direction described above.

The headset may comprise a plurality of side bands, wherein the second connector element of each side band is configured to be connected to the (e.g., same and/or only) first connector element such that the length of each of the plurality of side bands (e.g., between the respective first end and the first connector element) can be adjusted separately from the length of the others of the plurality of side bands. The plurality of side bands may comprise different second connector elements or similar second connector elements. For example, a number of or a distance between fixture elements of the second connector elements may differ between second connector elements of different side bands.

In a first variant, the first connector element comprises the at least one fixture counterpart. In this variant, the second connector element may comprise the plurality of fixture elements. The at least one fixture counterpart may be configured to couple to the one or more of the plurality of fixture elements of each of the plurality of side bands. For example, the pin may be configured to extend through the (e.g., through-)hole of each of the plurality of side bands.

In a second variant, the second connector element comprises the at least one fixture counterpart. In this variant, the first connector element may comprise the plurality of fixture elements. The at least one fixture counterpart of each of the plurality of side bands may be configured to (e.g., simultaneously) couple to the same of the one or more of the plurality of fixture elements.

The first connector element may be arranged on an element of the headband. The element of the headband may be configured to be placed in a temporal region of the user's head. In this context, the term "temporal" is to be understood as designating an anatomical location rather than being related to time.

The first end of the at least one side band may be attached relative to the headband. The first end of the at least one side band may be indirectly attached to the headband (e.g., via a connection portion), or the first end of the at least one side band may be directly attached to the headband.

The headset may further comprise a middle band attached (e.g., relative, indirectly or directly) to the headband. The middle band may be configured to extend between a front side and a rear side of the user's head, wherein the at least one side band is attached to the middle band. In this case, the middle band may be referred to as a connection portion. For example, the middle band essentially extends in or parallel to a sagittal plane of a user when the user is wearing the headset. The middle band may comprise a first longitudinal end (e.g., relative, indirectly or directly) attached to the headband and an opposite, second longitudinal end (e.g., relative, indirectly or directly) attached to the headband.

At least one of the headband, the at least one sideband and/or the middle band may (e.g., each) have a fixed length. The at least one sideband may have a fixed length between the first end and the second connector element. Alternatively or additionally, the headband, the at least one sideband and/or the middle band may be deformable, for example flexible, in length to a certain extent (e.g., above or equal to 0.1%, above or equal to 1%, below or equal to 5%, below or equal to 15%, for example from 0.1% to 15% or from 1% to 10%).

The headset may comprise at least one electrode receptacle. The at least one electrode receptacle may be arranged in a fixed spatial position relative to at least one of the headset, the headband, the at least one sideband and the middle band. The at least one electrode receptacle may be configured to (e.g., removably) hold the measurement electrode. The at least one electrode receptacle may be configured to couple to the measurement electrode. The at least one electrode contact may be arranged at, attached to or disposed on the at least one electrode receptacle. Each of the at least one electrode receptacle may be configured to hold a different one of a plurality of measurement electrodes. The at least one electrode receptacle may be arranged such that the measurement electrode is placed on the user's head at a position according to the 10-10 or 10-20 EEG electrode placement scheme when the user is wearing the headset.

The headset may further comprise a first circuit board. The first circuit board may be arranged at an element or portion of the headset to be placed on a rear side of the user's head. One or more wires electrically may connect the first circuit board with the at least one electrode contact.

The first circuit board may be arranged within a closable pocket attached to or formed by the headband. One or more of the at least one electrode contact may be arranged at (e.g., arranged on, embedded in or attached to) the middle band. The one or more wires may be attached (e.g., glued or stitched) to or embedded in the headband, the middle band and/or the sideband. The one or more wires may extend in a zig-zag pattern or a wave pattern. The one or more wires may be electrically shielded wires. The one or more wires may be covered with a (e.g., electrically shielding) protective cover.

The headset may further comprise a second circuit board comprising circuitry for collecting an electroencephalography, EEG, measurement signal from or via the first circuit board, wherein the second circuit board is arranged at the element or portion of the headset to be placed on the rear side of the user's (e.g., patient's or wearer's) head. The second circuit board may be removably coupled to the first circuit board. The second circuit board may be removably coupled to the first circuit board via an (e.g., push-in) electrical connector. The electrical connector may be a board-to-board connector or a printed circuit board, PCB, connector. The second circuit board may be arranged in the closable pocket together with the first circuit board.

According to a second aspect, an electroencephalography, EEG, measurement system is provided. The EEG measurement system comprises the headset according to the first aspect and at least one EEG measurement electrode. The at least one electrode contact is electrically connected to the at least one EEG measurement electrode.

The at least one EEG measurement electrode may be a dry electrode. The at least one EEG measurement electrode may comprise a plurality of electrode pins or fingers. The EEG measurement electrode may be removably held by the electrode receptacle described above. The EEG measurement electrode may made from an electrically conductive elastic material such as a thermoelastic polymer doped with silver or silver chloride. The system may be configured such that the electrode pins or fingers are placed on the scalp of a user (e.g., at a position according to the 10-10 or 10-20 EEG electrode placement scheme) when the user is wearing the headset.

### Brief Description of the Drawings

Further details, advantages and aspects of the present disclosure will become apparent from the following embodiments taken in conjunction with the drawings, wherein:
- Fig. 1: shows a first embodiment of a system in accordance with the present disclosure;
- Fig. 2: shows the 10-10 EEG electrode placement scheme;
- Fig. 3: shows the system of Fig. 1 during assembly;
- Fig. 4: shows a first detail view of a portion of the system of Fig. 1;
- Fig. 5: shows a second detail view of a portion of the system of Fig. 1;
- Fig. 6: shows the system of Fig. 1 during assembly;
- Fig. 7: shows components of the system of Fig. 1;
- Fig. 8: shows a detail view of a rear side of the system of Fig. 1; and
- Fig. 9: shows a further detail view of a rear side of the system of Fig. 1.

### Detailed Description

In the following description, exemplary embodiments of a size-adjustable headset and an EEG measurement system will be explained with reference to the drawings. The same reference numerals will be used to denote the same or similar structural features.

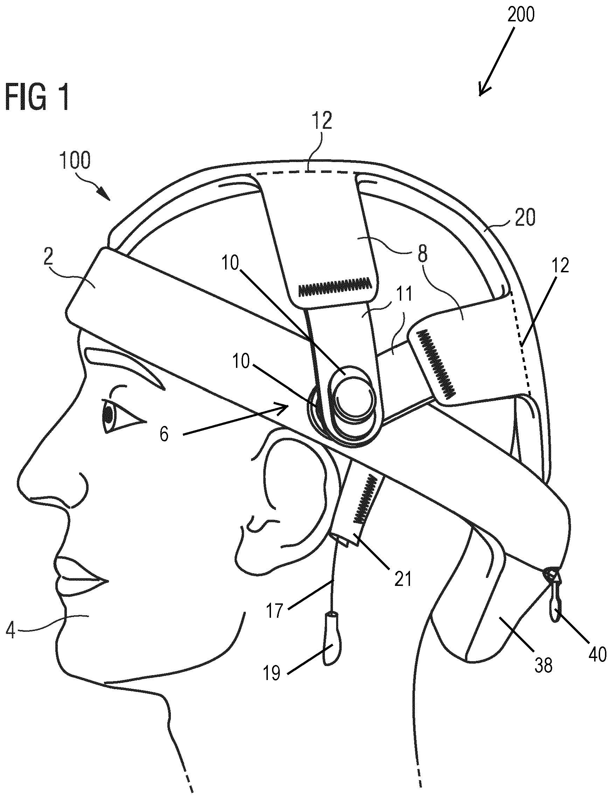

Fig. 1 shows a first embodiment of a system 200 in accordance with the present disclosure. The system 200 comprises a size-adjustable headset 100 for monitoring bioelectric signals. The headset 100 comprises a headband 2. The headband can be configured to be put on or around a user's head 4. The headband 2 may be configured to encircle at least a portion of the user's head 4 when the headset 100 is worn by the user. The headband 2 comprises a first connector element 6 that may lie in a temporal region of the user's head 4.

The headset 100 further comprises at least one sideband 8 having a first end 12. The first end 12 may be a longitudinal end of the at least one sideband 8 and may be connected to a middle band 20 of the headset 100. The at least one side band 8 comprises a second connector element 10. The second connector element 10 may be arranged on an end segment 11 of the at least one sideband 8. The second connector element 10 may thereby be arranged at a longitudinal end of the at least one sideband 8 that is opposite to the first end 12 of the at least one sideband 8. The at least one side band 8 may extend between the middle band 20 and the second connector element 10. The headset 100 may have a symmetrical shape.

In the illustrated example, the headset 100 comprises four side bands 8, two of which are connected to a first connector element 6 at a left side of the user's head 4, and the remaining two of which (not visible in Fig. 1) are connected to another connector element 6 (not visible in Fig. 1) at a right side of the user's head 4.

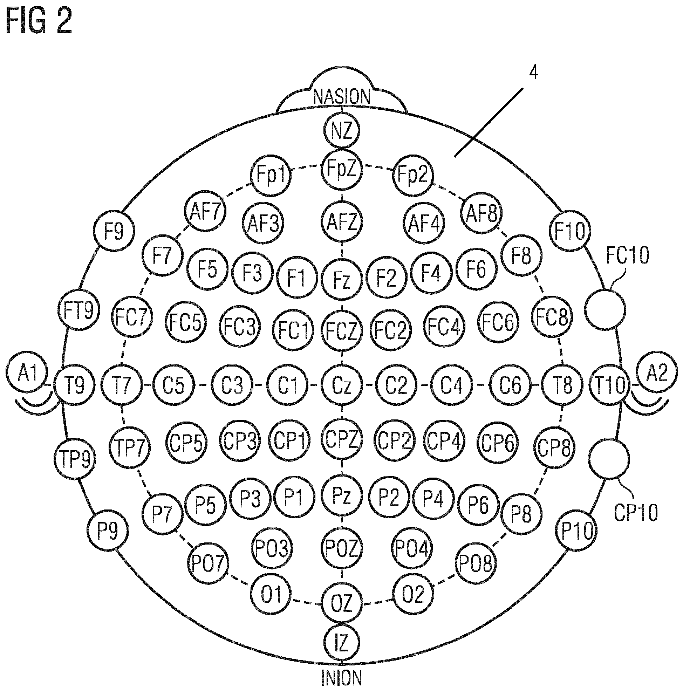

Fig. 2 shows the 10-10 EEG electrode placement scheme. The headset 100 may be configured such that the middle band 20 extends essentially across the positions Oz, Pz, Cz, Fz and Fpz. That is, the middle band 20 may extend in a sagittal plane along a center line of the user's head 4. The headband 2 may extend across the positions Fpz, Oz, T7 and T8. It is noted that in the 10-20 EEG electrode placement scheme, the positions T7 and T8 shown in Fig. 2 are usually referred to as T3 and T4. The at least one side band 8 may extend across one or more of the positions AF3, AF4, F1-F6, FC1-FC6, C1-C6, CP1-CP6, P1-P6, Po3 and Po4. In the example shown in Fig. 1, one of the side bands 8 extends across the position P3 and the other extends across the position FC3.

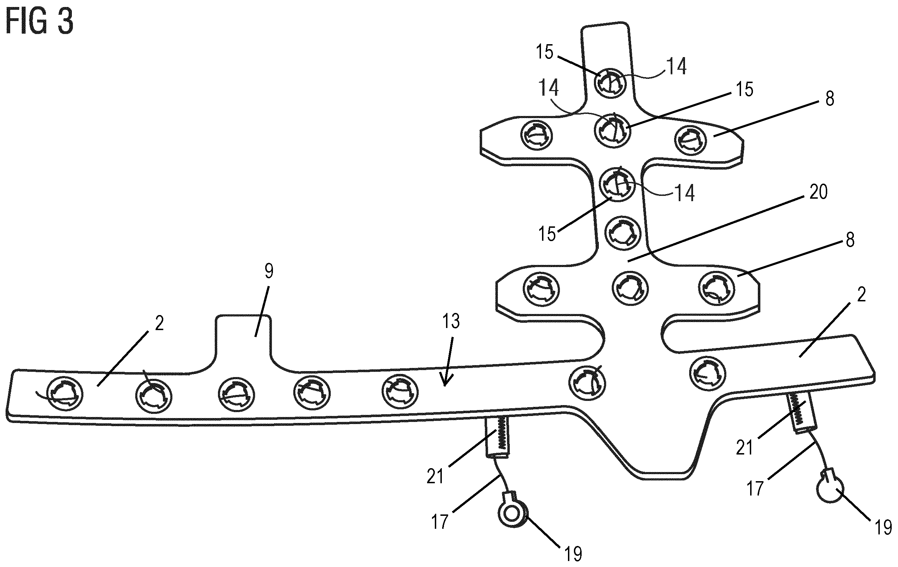

Fig. 3 shows the system 200 of Fig. 1 during assembly. To obtain the headset 100 shown in Fig. 1, the headband 2 illustrated in Fig. 3 may be sewn, glued or otherwise attached together to form an overall circle-shape, and the middle band 20 may be sewn, glued or otherwise attached to a flap 9 extending from the headband 2. Additionally, the first connector elements 6 may be connected to the second connector elements.

In Fig. 3, a bottom or inner surface 13 of the headset 100 is shown, which surface 13 faces the scalp of the user when the user is wearing the assembled headset 100. As can be seen, the headset 100 comprises at least one electrode contact 14 configured to be electrically connected to a measurement electrode. Each electrode contact 14 may be arranged at an electrode receptacle 15, wherein the individual electrode receptacles 15 may be arranged at the headband 2, the at least one sideband 8 or the middle band 20. Each electrode receptacle 15 may be configured to removably hold a measurement electrode. The headset 100 may comprise two electrode contacts 17, each being attached to a reference electrode 19 which may be adhesively mounted on a portion of the user's head 4. The electrode contacts 17 may be partially housed within a protective sleeve 21. The receptacles 15 may be arranged such that each measurement electrode is placed on one of the aforementioned positions of the 10-10 or 10-20 EEG electrode placement scheme when the electrode receptacles 15 hold the electrodes and the user is wearing the headset 100.

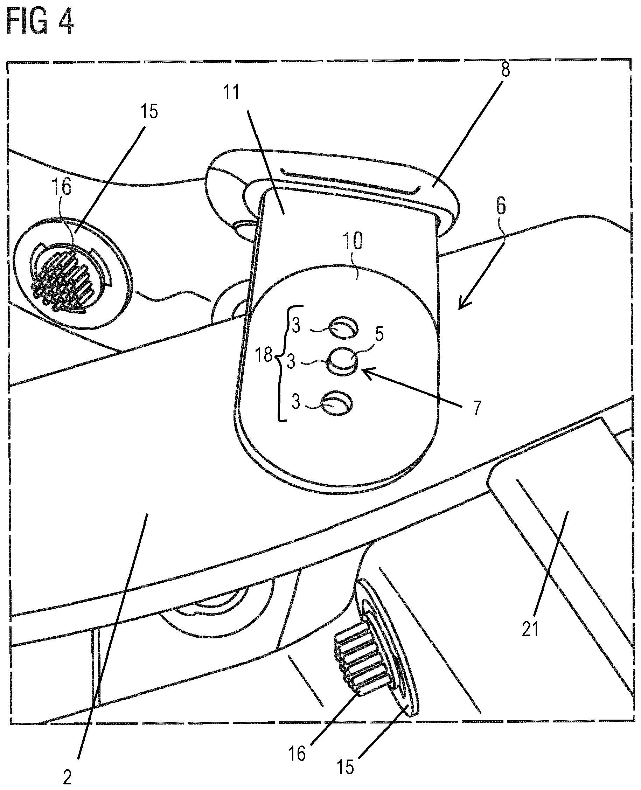

Fig. 4 shows a first detail view of a portion of the system 200 of Fig. 1. The system 200 comprises at least one EEG measurement electrode 16. The at least one electrode contact 14 is electrically connected to the at least one EEG measurement electrode 16. The electrode 16 may be a wet electrode or a dry electrode having a plurality of conductive flexible electrode fingers or pins configured to contact a surface of the user's head 4. In a preferred embodiment, the electrode 16 is a dry electrode. The electrode 16 may be removably held by one of the receptacles 15 of the headset 100.

The first connector element 6 and the second connector element 10 are configured to be connected to one another such that a length (or distance) of the at least one side band 8 between the first end 12 and the first connector element 6 can be adjusted. The first and second connector elements 6, 10 may be configured such that the length of the at least one side band 8 can be adjusted between a plurality of predefined lengths. To this end, the first connector element 6 and the second connector element 10 may be configured to be connected to one another in a plurality of predefined positions. The positions may be relative to each other.

According to a first configuration, the second connector element 10 may comprise a plurality of fixture elements 18 spaced apart from one another in a (e.g., lengthwise or longitudinal) direction of the at least one side band 8. In this case, the first connector element 6 may comprise at least one fixture counterpart 7 configured to couple to one or more of the plurality of fixture elements 18 at a time. For instance, in compliance with Fig. 4, the plurality of fixture elements 18 of the second connector element 10 may comprise at least one through-hole 3 extending essentially orthogonal to an outer surface of the at least one sideband 8 and an outer surface of the headband 2. The at least one fixture counterpart 7 of the first connector element 6 may comprise a pin 5 configured to extend in a similar direction as the through-hole 3. The pin 5 may fully extend through the through-hole 3 and emerge on an opposite side of the sideband 8. The through-holes 3 may each define a spatial position for the at least one sideband 8 relative to the headband 2. The pin 5 may be essentially cylindrical and comprise a broadened head portion or end element.

According to a second configuration, the first connector element 6 may comprise a plurality of fixture elements spaced apart from one another in a (e.g., lengthwise or longitudinal) direction of the at least one side band 8. In this case, the second connector element 10 may comprise at least one fixture counterpart configured to couple to one or more of the plurality of fixture elements at a time. For instance, the plurality of fixture elements of the first connector element 6 may comprise a plurality of pins, each extending essentially orthogonal to an outer surface of the headband 2. The at least one fixture counterpart of the second connector element 10 may comprise at least one through-hole configured to extend in a similar direction as the pins. Each of the pins may be configured to fully extend through the through-hole when aligning the at least one sideband 8 with respect to the headband 2 accordingly. The pins may each define a spatial position for the at least one sideband 8 relative to the headband 2. The pins may each be essentially cylindrical and comprise a broadened head portion or end element.

In both configurations, each pin may comprise a broadened head portion or an angled distal end section. The diameter of the body of each pin may be identical to that of the through-holes, whereas the diameter of the broadened head portion or angled distal end section may be greater than that of the through-holes. The broadened head portion or angled distal end section may be sized to prevent unintentional decoupling of the pin and the through-hole, while allowing disconnection of the pin from the through-hole by the user.

In a first variation, the plurality of fixture elements 18 may consist of a plurality of holes 3 and the at least one fixture counterpart 7 may consist of a single pin 5. This may enable a variation in angle between the at least one sideband 8 and the headband 2, even when the first connector element 6 is connected to the second connector element 10.

In a second variation, the at least one fixture counterpart 7 may comprise a plurality of pins 5 and the plurality of fixture elements 18 may comprise a plurality of holes 3. Each of the plurality of pins may be configured to couple with a different hole 3 of the plurality of fixture elements 18 at a given time. This may enable a more rigid connection between the first connector element 6 and the second connector element 10.

The first variation and the second variation may each be combined with the first configuration and/or the second configuration. The plurality of fixture elements 18 may generally be considered as female coupling elements, while the fixture counter parts 7 may generally be as a male coupling element. It is to be noted that the plurality of fixture elements 18 is not limited to holes and the at least one fixture counterpart 7 is not limited to a pin. Other variants of these two coupling components 7, 18 are possible. For instance, the plurality of fixture elements 18 may comprise a catch and the at least one fixture counterpart 7 may comprise a bolt configured to couple with the catch. It is noted that other connector mechanisms, such as a velcro fastener mechanism, a snap-fit coupling, magnetic connectors, etc. are also conceivable. In a preferred embodiment, the connection mechanism comprises a pin and a hole.

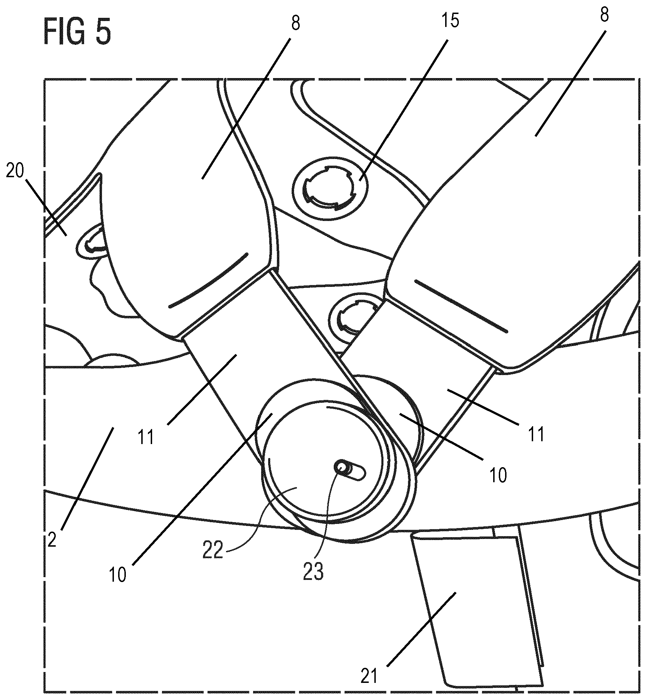

A plurality of second connector elements 10 of multiple sidebands 8 may be stacked on top of each other over the first connector element 6, as for instance shown in Figs. 1 and 5. The first connector element 6 may be configured to simultaneously connect to each of the plurality of second connector elements 10. This may enable adjusting the length of each of the side bands 8 individually, using the single first connector element 6. An example of such a configuration is illustrated in Fig. 5, which shows a second detail view of a portion of the system 200 of Fig. 1. A length and shape of the pin 5 may be chosen such that it can simultaneously extend through holes 3 of multiple second connector elements 10 of a plurality of sidebands 8.

The headset 100 may further comprise a protection unit 22 configured to, when the first connector element 6 and the second connector element 10 are connected to one another and when the protection unit 22 is in a first state, couple to the pin 5 to restrict movement of the hole 3 relative to the pin 5 in an axial direction of the pin 5. The protection unit 22 may be coupled to the broadened head (e.g., end element) of the pin 5.

The protection unit 22 may block a disconnection of the first connector element 6 and the second connector element 10. The protection unit 22 may further be configured to be shifted to a second state in which it allows the disconnection of the first connector element 6 and the second connector element 10. To this end, the protection unit 22 may comprise a key mechanism 23 configured to, when actuated by a key, shift the protection unit 22 from the first state to the second state and/or from the second state to the first state.

The protection unit 22 may comprise a cap or cover configured to be put on or over (e.g., proximal to) the first connector element 6 and the second connector element 10.

As noted above, the protection unit 22 may comprise a key mechanism 23 configured to, when actuated by a key, shift the protection unit from the first state to the second state and/or from the second state to the first state. In an alternative, the key mechanism 23 may only be configured to, when actuated by a key, shift the protection unit 22 from one state to the other, while not requiring any actuation by a key in the reverse direction. For instance, the key mechanism 23 may be used to shift the protection unit 22 from the first state to the second state and allow disconnection of the first connector element 6 and the second connector element 10, while the protection unit 22 may automatically, without requiring any actuation by a key mechanism 23, shift from the second state to the first state to block disconnection, once the protection unit 22 is put on or over the first connector element 6 and the second connector element 10 and/or coupled to the pin 5.

In the shown example, the key mechanism 23 can be actuated by linearly sliding a locking element using a pin-shaped key. This may unlock and decouple the protection unit 22 from the broadened head of the pin 5, thereby allowing removing the protection unit 22 from the pin 5, and allowing subsequently removing the sidebands 8 from the pin. Other variants of the protection unit 22 are possible, as long as the above functionality of the protection unit 22 is ensured.

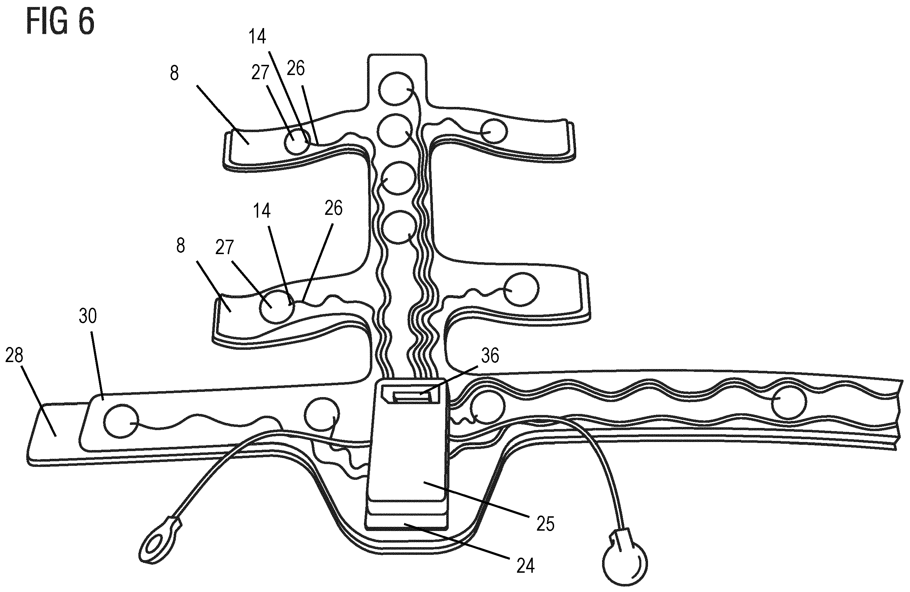

Fig. 6 shows the system 200 of Fig. 1 at an even earlier stage of assembly than illustrated in Fig. 3. In Fig. 6, the opposite side of the headset 100 is shown, as compared to Fig. 3. As can be seen in Fig. 6, the headset 100 may comprise a first circuit board 24 arranged at an element or portion of the headset 100, which may be placed on a rear side of the user's head 4. The first circuit board 24 may be partially covered by a protective housing 25. The headset 100 may further comprise a plurality of wires 26, each electrically connecting the first circuit board 24 with one of the electrode contacts 14.

Each electrode contact 14 may be electrically connected to one of the receptacles 15 and/or one of the EEG measurement electrodes 16. In the example illustrated in Fig. 6, the headband 2, the middle band 20 and the sidebands 8 are formed of a plurality of material layers, comprising an outer layer 28 and an inner layer 30. Additional layers may also be provided. The plurality of layers may comprise one or more layers made of a hydrophilic, non-swelling and/or biocompatible foam. The plurality of layers may comprise one or more layers made from a knitted fabric such as a 4-way stretch synthetic knitted fabric. To obtain the headset as illustrated in Figs. 1 and 3, the plurality of material layers may be wrapped by a protective cover material, and the receptacles 15 may be inserted into cavities 27 or through-holes 27 in at least some of the plurality of layers. The protective cover material may be a (e.g., 4-way stretch) knitted fabric with a water-resistant coating.

The wires 26 may also be wrapped with or covered by the protective cover material, thereby being embedded within the headset 100. The wires 26 may be sewn, glued, clipped or attached with similar means onto the inner layer 30. The wires 26 may be electrically shielded wires. The wires 26 in the illustrated example form a wave pattern. The arrangement of the wires 26 may decrease an influence of external electromagnetic noise on a measurement obtained via the electrodes 16.

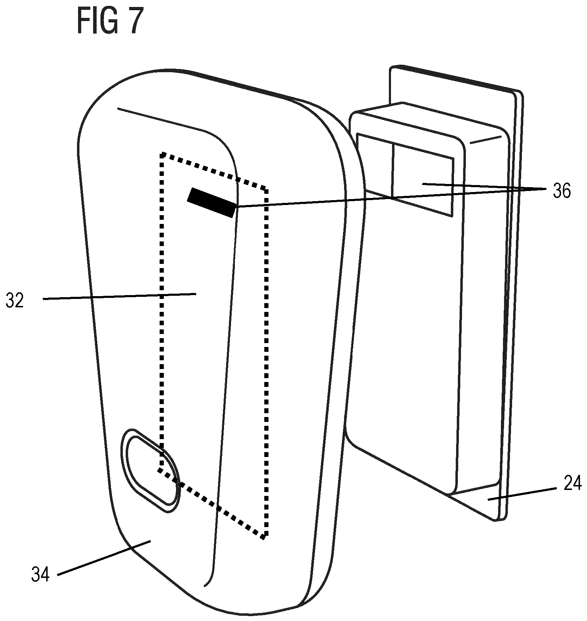

Fig. 7 shows components of the system 200 of Fig. 1. The headset 100 may further comprise a second circuit board 32. The second circuit board 32 may comprise circuitry for collecting an electroencephalography, EEG, measurement signal from the first circuit board 24. The second circuit board 32 may be arranged inside a protective housing 34 and may be arranged at the element or portion of the headset 100 to be placed on the rear side of the wearer's head 4. The second circuit board 32 may be removably coupled to the first circuit board 24, for example via a board-to-board connector 36. The second circuit board 32 may comprise a memory configured to store the EEG measurement or diagnostic data obtained by processing the EEG measurement. The first circuit board 24 may not store the EEG measurement or diagnostic data obtained by processing the EEG measurement.

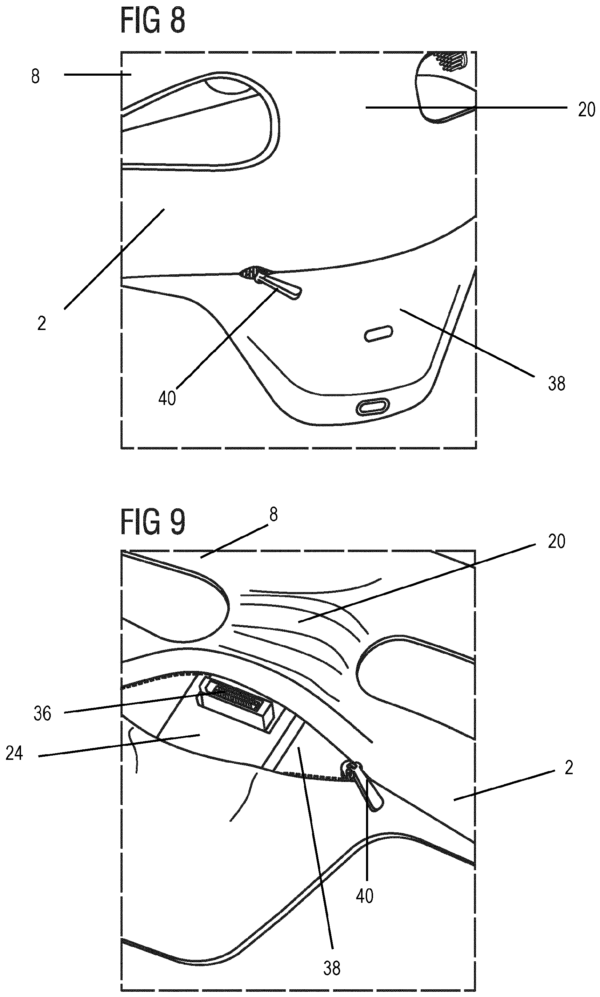

Fig. 8 shows a detail view of a rear side of the system 200 of Fig. 1. As can be seen, a pocket 38 may be formed, for instance at the element or portion of the headset 100 to be placed on the rear side of the patient's head 4. The pocked 38 may be closable via a zip fastener 40. Both the first circuit board 24 and the second circuit board 32, together with the respective protective housings 25, 34, may be arranged inside the pocket 38.

Fig. 9 shows a further detail view of a rear side of the system 200 of Fig. 1. Compared with Fig. 8, the zip fastener 40 has been opened, the second circuit board 32 together with protective housing 34 has been decoupled from the first circuit board 24 and removed from the pocket 38.

As apparent from the above, an advantageous size-adjustable headset and EEG measurement system are provided. The size adjustment may allow fitting the headset 100 to different head sizes and head shapes, thus allowing to produce headsets and systems that can be used by a broad variety of users. The size adjustment ensures a comfortable fit and a reliable and repeatable positioning of measurement electrodes, for instance on the 10-10 or 10-20 placement positions whilst enabling an easy and fast adjustment of the headset 100.

The system 200 described herein may be manufactured at low cost compared with existing EEG headsets.

The protection unit 22 may prevent an end user of the headset 100 from changing the size, whilst allowing authorized personnel having a matching key to do so with little effort. Since the end user cannot change the size, it can be ensured that the electrodes remain at the same position for consecutive measurements, thus enabling the measurement of consistent data sets. Furthermore, the headset 100 can be given to the user for use at home, while ensuring that the headset 100 and resulting measurements cannot be manipulated. As opposed thereto, common headsets may only be used in clinical, monitored environments, where it can be assured that the user has not changed or manipulated the size of the headset. This prevention of the unauthorized changing of the size thus ensures that a consistent set of data can be measured.

Removably holding the electrodes may allow replacement thereof in case of damage or for cleaning purposes.

The arrangement of the wires 26 described above may decrease an influence of external electromagnetic noise on a measurement obtained via the electrodes 16.

The second circuit board 32 described above may be replaced by authorized (e.g., clinical) personnel, allowing for a quick re-use of the headset 100 without requiring a potentially time-consuming data transferal process of stored EEG recordings.

The second circuit board 32 described above may be easily replaced with a new version in case of hardware or software updates.

Arranging the first and second circuit board 24, 32 at the rear side of the user's head may improve fit of the headset 100 and comfort of the user.

Alternative and/or additional advantages may be obtained with the subject matter disclosed herein. The invention is defined by the appended claims.

## Claims

1. A size-adjustable headset (100) for monitoring bioelectrical signals, the headset (100) comprising:
a headband (2) comprising a first connector element (6);
at least one side band (8) comprising a second connector element (10) distant from a first end (12) of the at least one side band (8); and
at least one electrode contact (14) configured to be electrically connected to a measurement electrode (16),
wherein the first connector element (6) and the second connector element (10) are configured to be connected to one another such that a length of the at least one side band (8) between the first end (12) and the first connector element (6) can be adjusted,
**characterized in that**:
the headset (100) further comprising a protection unit (22) configured to, when the first connector element (6) and the second connector element (10) are connected to one another and when the protection unit (22) is in a first state, block a disconnection of the first connector element (6) and the second connector element (10),
the protection unit (22) is configured to, when the first connector element (6) and the second connector element (10) are connected to one another and when the protection unit (22) is in a second state, allow the disconnection of the first connector element (6) and the second connector element (10),
the protection unit (22) comprises a key mechanism (23) configured to, when actuated by a key, shift the protection unit (22) from the first state to the second state and/or from the second state to the first state.

2. The headset (100) of claim 1, wherein the first connector element (6) and the second connector element (10) are configured to be connected to one another such that the length of the at least one side band (8) between the first end (12) and the first connector element (10) can be adjusted between a plurality of predefined lengths; and/or
wherein the first connector element (6) and the second connector element (10) are configured to be connected to one another in a plurality of predefined positions.

3. The headset (100) of any one of claims 1 or 2, wherein one of the first connector element (6) and the second connector element (10) comprises a plurality of fixture elements (18) spaced apart from one another in a direction of the at least one side band (8), and wherein the respective other of the first connector element (6) and the second connector element (10) comprises at least one fixture counterpart (7) configured to couple to one or more of the plurality of fixture elements (18).

4. The headset (100) of claim 3, wherein the plurality of fixture elements (18) comprises a hole (3) and the at least one fixture counterpart (7) comprises a pin (5) configured to at least partially extend into the hole (3).

5. The headset (100) of claim 4, wherein the hole (3) is a through-hole, wherein the pin (5) is configured to extend into the hole (3) from a first side of the hole (3), and wherein the pin (5) is configured to protrude from the hole (3) from a second side of the hole (3), wherein the second side is opposite to the first side.

6. The headset (100) of claim 4 or 5,
wherein the hole (3) extends in a lateral direction of the at least one sideband (8) and the pin (5) extends in a lateral direction of the headband (2), and/or
wherein the pin (5) extends in a lateral direction of the at least one sideband (8) and the hole (3) extends in a lateral direction of the headband (2).

7. The headset (100) of any one of claims 4 to 6, wherein the pin (5) comprises a first axial end and a second axial end, the first axial end being closer to the headband (2) than the second axial end, wherein the pin (5) comprises an end element at the second axial end, the end element being radially wider than a part of the pin (5) adjacent to the end element.

8. The headset (100) of claim 7 as far as depending on claim 1, wherein the protection unit (22) is configured to, in the first state, couple to the end element of the pin (5) to restrict movement of the hole (3) relative to the pin (5) in an axial direction of the pin (5).

9. The headset (100) of any one of claims 1 to 8, wherein the headset (100) comprises a plurality of side bands (8), wherein the second connector element (10) of each side band is configured to be connected to the first connector element (6) such that the length of each of the plurality of side bands (8) can be adjusted separately from the length of the others of the plurality of side bands (8).

10. The headset (100) of claim 9 as far as depending on claim 3, wherein the first connector element (6) comprises the at least one fixture counterpart (7), wherein the at least one fixture counterpart (7) is configured to couple to the one or more of the plurality of fixture elements (18) of each of the plurality of side bands (8).

11. The headset (100) of claim 9 or 10 as far as depending on any one of claims 5 to 8, wherein the pin (5) is configured to extend through the through-hole (3) of each of the plurality of side bands (8).

12. The headset (100) of any one of claims 1 to 8, wherein the first connector element (6) is arranged on an element of the headband (2) configured to be placed in a temporal region of a user's head (4); and/or
wherein the first end (12) of the at least one side band (8) is attached relative to the headband (2).

13. The headset (100) of any one of claims 1 to 12, further comprising a middle band (20) attached to the headband (2), wherein the middle band (20) is configured to extend between a front side and a rear side of the user's head (4), wherein the at least one side band (8) is attached to the middle band (20).

14. The headset (100) of any one of claims 1 to 11, further comprising:
a first circuit board (24); and
one or more wires (26) electrically connecting the first circuit board (24) with the at least one electrode contact (14); and/or
the headset (100) further comprising:
a second circuit board (32) comprising circuitry for collecting an electroencephalography measurement signal from the first circuit board (24),
wherein the second circuit board (32) is removably coupled to the first circuit board (24).

15. An electroencephalography, EEG, measurement system (200) comprising:
the headset (100) according to any one of claims 1 to 14; and
at least one EEG measurement electrode (16),
wherein the at least one electrode contact (14) is electrically connected to the at least one EEG measurement electrode (16).

## Patentansprüche

1. Größenverstellbares Headset (100) zum Überwachen bioelektrischer Signale, das Headset (100) umfassend:
ein Kopfband (2), umfassend ein erstes Verbindungselement (6);
mindestens ein Seitenband (8), umfassend ein zweites Verbindungselement (10), das von einem ersten Ende (12) des mindestens einen Seitenbands (8) beabstandet ist; und
mindestens einen Elektrodenkontakt (14), der dazu ausgebildet ist, elektrisch mit einer Messelektrode (16) verbunden zu sein,
wobei das erste Verbindungselement (6) und das zweite Verbindungselement (10) zum Verbinden miteinander ausgebildet sind, sodass eine Länge des mindestens einen Seitenbands (8) zwischen dem ersten Ende (12) und dem ersten Verbindungselement (6) einstellbar ist,
**dadurch gekennzeichnet, dass**:
das Headset (100) weiter eine Schutzeinheit (22) umfasst, die dazu ausgebildet ist, dass sie, wenn das erste Verbindungselement (6) und das zweite Verbindungselement (10) miteinander verbunden sind und wenn sich die Schutzeinheit (22) in einem ersten Zustand befindet, ein Trennen des ersten Verbindungselements (6) und des zweiten Verbindungselements (10) blockiert,
die Schutzeinheit (22) dazu ausgebildet ist, dass sie, wenn das erste Verbindungselement (6) und das zweite Verbindungselement (10) miteinander verbunden sind und wenn sich die Schutzeinheit (22) in einem zweiten Zustand befindet, das Trennen des ersten Verbindungselements (6) und des zweiten Verbindungselements (10) ermöglicht,
die Schutzeinheit (22) einen Schlüsselmechanismus (23) umfasst, der dazu ausgebildet ist, dass er bei Betätigung durch einen Schlüssel die Schutzeinheit (22) aus dem ersten Zustand in den zweiten Zustand und/oder aus dem zweiten Zustand in den ersten Zustand wechselt.

2. Headset (100) nach Anspruch 1, wobei das erste Verbindungselement (6) und das zweite Verbindungselement (10) dazu ausgebildet sind, dass sie so miteinander verbindbar sind, dass die Länge des mindestens einen Seitenbands (8) zwischen dem ersten Ende (12) und dem ersten Verbindungselement (10) zwischen einer Vielzahl an vordefinierten Längen einstellbar ist; und/oder
wobei das erste Verbindungselement (6) und das zweite Verbindungselement (10) dazu ausgebildet sind, in einer Vielzahl von vordefinierten Positionen miteinander verbindbar zu sein.

3. Headset (100) nach einem der Ansprüche 1 oder 2, wobei eines des ersten Verbindungselements (6) und des zweiten Verbindungselements (10) eine Vielzahl an Befestigungselementen (18) umfasst, die in einer Richtung des mindestens einen Seitenbands (8) voneinander beabstandet sind, und wobei das jeweils andere des ersten Verbindungselements (6) und des zweiten Verbindungselements (10) mindestens ein Befestigungsgegenstück (7) umfasst, das zum Koppen mit einem oder mehreren der Vielzahl an Befestigungselementen (18) ausgebildet ist.

4. Headset (100) nach Anspruch 3, wobei die Vielzahl an Befestigungselementen (18) ein Loch (3) umfassen und das mindestens eine Befestigungsgegenstück (7) einen Stift (5) umfasst, der zum mindestens teilweisen Erstrecken in das Loch (3) ausgebildet ist.

5. Headset (100) nach Anspruch 4, wobei das Loch (3) ein Durchgangsloch ist, wobei der Stift (5) dazu ausgebildet ist, sich von einer ersten Seite des Lochs (3) in das Loch (3) zu erstrecken, und wobei der Stift (5) dazu ausgebildet ist, von einer zweiten Seite des Lochs (3) aus dem Loch (3) herauszuragen, wobei die zweite Seite der ersten Seite gegenüberliegt.

6. Headset (100) nach Anspruch 4 oder 5,
wobei sich das Loch (3) in einer seitlichen Richtung des mindestens einen Seitenbands (8) erstreckt und sich der Stift (5) in einer seitlichen Richtung des Kopfbands (2) erstreckt, und/oder
wobei sich der Stift (5) in einer seitlichen Richtung des mindestens einen Seitenbands (8) erstreckt und sich das Loch (3) in einer seitlichen Richtung des Kopfbandes (2) erstreckt.

7. Headset (100) nach einem der Ansprüche 4 bis 6, wobei der Stift (5) ein erstes axiales Ende und ein zweites axiales Ende umfasst, wobei das erste axiale Ende näher an dem Kopfband (2) ist als das zweite axiale Ende, wobei der Stift (5) ein Endelement an dem zweiten axialen Ende umfasst, wobei das Endelement radial breiter ist als ein zu dem Endelement benachbarter Teil des Stifts (5).

8. Headset (100) nach Anspruch 7, soweit es von Anspruch 1 abhängt, wobei die Schutzeinheit (22) in dem ersten Zustand zum Koppeln mit dem Endelement des Stifts (5) ausgebildet ist, um die Bewegung des Lochs (3) relativ zu dem Stift (5) in einer axialen Richtung des Stifts (5) zu begrenzen.

9. Headset (100) nach einem der Ansprüche 1 bis 8, wobei das Headset (100) eine Vielzahl an Seitenbändern (8) umfasst, wobei das zweite Verbindungselement (10) jedes Seitenbands zum Verbinden mit dem ersten Verbindungselement (6) ausgebildet ist, sodass die Länge jedes der Vielzahl an Seitenbändern (8) separat von der Länge der anderen der Vielzahl an Seitenbändern (8) einstellbar ist.

10. Headset (100) nach Anspruch 9, soweit es von Anspruch 3 abhängt, wobei das erste Verbindungselement (6) das mindestens eine Befestigungsgegenstück (7) umfasst, wobei das mindestens eine Befestigungsgegenstück (7) zum Koppeln mit dem einen oder den mehreren der Vielzahl an Befestigungselementen (18) jedes der Vielzahl an Seitenbändern (8) ausgebildet ist.

11. Headset (100) nach Anspruch 9 oder 10, soweit es von einem der Ansprüche 5 bis 8 abhängt, wobei der Stift (5) dazu ausgebildet ist, sich durch das Durchgangsloch (3) jedes der Vielzahl an Seitenbändern (8) zu erstrecken.

12. Headset (100) nach einem der Ansprüche 1 bis 8, wobei das erste Verbindungselement (6) an einem Element des Kopfbands (2) angeordnet ist, das dazu ausgebildet ist, dass es in einem Schläfenbereich des Kopfes (4) eines Benutzers platzierbar ist; und/oder
wobei das erste Ende (12) des mindestens einen Seitenbands (8) relativ zu dem Kopfband (2) befestigt ist.

13. Headset (100) nach einem der Ansprüche 1 bis 12, ferner umfassend ein Mittelband (20), das an dem Kopfband (2) befestigt ist, wobei das Mittelband (20) dazu ausgebildet ist, sich zwischen einer Vorderseite und einer Rückseite des Kopfes (4) des Benutzers zu erstrecken, wobei das mindestens eine Seitenband (8) an dem Mittelband (20) befestigt ist.

14. Headset (100) nach einem der Ansprüche 1 bis 11, ferner umfassend:
eine erste Leiterplatte (24); und
einen oder mehrere Drähte (26), die die erste Leiterplatte (24) mit dem mindestens einen Elektrodenkontakt (14) elektrisch verbinden; und/oder
das Headset (100) ferner umfassend:
eine zweite Leiterplatte (32), umfassend eine Schaltung zum Sammeln eines Elektroenzephalographie-Messsignals von der ersten Leiterplatte (24),
wobei die zweite Leiterplatte (32) lösbar mit der ersten Leiterplatte (24) gekoppelt ist.

15. Elektroenzephalographie-, EEG-Messsystem (200), umfassend:
das Headset (100) nach einem der Ansprüche 1 bis 14; und
mindestens eine EEG-Messelektrode (16),
wobei der mindestens eine Elektrodenkontakt (14) elektrisch mit der mindestens einen EEG-Messelektrode (16) verbunden ist.

## Revendications

1. Casque à taille ajustable (100) pour la surveillance de signaux bioélectriques, le casque (100) comprenant :
un serre-tête (2) comprenant un premier élément de connexion (6) ;
au moins une bande latérale (8) comprenant un deuxième élément de connexion (10) distant d'une première extrémité (12) de l'au moins une bande latérale (8) ; et
au moins un contact d'électrode (14) configuré pour être connecté électriquement à une électrode de mesure (16),
le premier élément de connexion (6) et le deuxième élément de connexion (10) étant configurés pour être connectés l'un à l'autre de manière à ce qu'une longueur de l'au moins une bande latérale (8) entre la première extrémité (12) et le premier élément de connexion (6) puisse être ajustée,
**caractérisé en ce que** :
le casque (100) comprend en outre une unité de protection (22) configurée pour, lorsque le premier élément de connexion (6) et le deuxième élément de connexion (10) sont connectés l'un à l'autre et lorsque l'unité de protection (22) est dans un premier état, bloquer une déconnexion du premier élément de connexion (6) et du deuxième élément de connexion (10),
l'unité de protection (22) est configurée pour, lorsque le premier élément de connexion (6) et le deuxième élément de connexion (10) sont connectés l'un à l'autre et lorsque l'unité de protection (22) est dans un deuxième état, permettre la déconnexion du premier élément de connexion (6) et du deuxième élément de connexion (10),
l'unité de protection (22) comprend un mécanisme à clé (23) configuré pour, lorsqu'il est actionné par une clé, faire passer l'unité de protection (22) du premier état au deuxième état et/ou du deuxième état au premier état.

2. Casque (100) selon la revendication 1, le premier élément de connexion (6) et le deuxième élément de connexion (10) étant configurés pour être connectés l'un à l'autre de manière à ce que la longueur de l'au moins une bande latérale (8) entre la première extrémité (12) et le premier élément de connexion (10) puisse être ajustée entre une pluralité de longueurs prédéfinies ; et/ou,
le premier élément de connexion (6) et le deuxième élément de connexion (10) étant configurés pour être connectés l'un à l'autre en une pluralité de positions prédéfinies.

3. Casque (100) selon l'une des revendications 1 ou 2, l'un parmi le premier élément de connexion (6) et le deuxième élément de connexion (10) comprenant une pluralité d'éléments fixes (18) espacés les uns des autres dans une direction de l'au moins une bande latérale (8), et l'autre respectif parmi le premier élément de connexion (6) et le deuxième élément de connexion (10) comprenant au moins un élément homologue fixe (7) configuré pour se coupler à un ou plusieurs de la pluralité d'éléments fixes (18).

4. Casque (100) selon la revendication 3, la pluralité d'éléments fixes (18) comportant un trou (3) et l'au moins un élément homologue fixe (7) comprenant une broche (5) configurée pour s'étendre au moins partiellement dans le trou (3).

5. Casque (100) selon la revendication 4, le trou (3) étant un trou traversant, la broche (5) étant configurée pour s'étendre dans le trou (3) à partir d'un premier côté du trou (3), et la broche (5) étant configurée pour faire saillie du trou (3) à partir d'un deuxième côté du trou (3), le deuxième côté étant opposé au premier côté.

6. Casque (100) selon la revendication 4 ou 5,
le trou (3) s'étendant dans une direction latérale de l'au moins une bande latérale (8) et la broche (5) s'étendant dans une direction latérale du serre-tête (2), et/ou
la broche (5) s'étendant dans une direction latérale de l'au moins une bande latérale (8) et le trou (3) s'étendant dans une direction latérale du serre-tête (2).

7. Casque (100) selon l'une des revendications 4 à 6, la broche (5) comprenant une première extrémité axiale et une deuxième extrémité axiale, la première extrémité axiale étant plus proche du serre-tête (2) que la deuxième extrémité axiale, la broche (5) comprenant un élément d'extrémité à la deuxième extrémité axiale, l'élément d'extrémité étant radialement plus large qu'une partie de la broche (5) adjacente à l'élément d'extrémité.

8. Casque (100) selon la revendication 7 dans la mesure où elle dépend de la revendication 1, l'unité de protection (22) étant configurée pour, dans le premier état, se coupler à l'élément d'extrémité de la broche (5) afin de restreindre le mouvement du trou (3) par rapport à la broche (5) dans une direction axiale de la broche (5).

9. Casque (100) selon l'une des revendications 1 à 8, le casque (100) comprenant une pluralité de bandes latérales (8), le deuxième élément de connexion (10) de chaque bande latérale étant configuré pour être connecté au premier élément de connexion (6) de manière à ce que la longueur de chacune de la pluralité de bandes latérales (8) puisse être ajustée séparément de la longueur des autres bandes de la pluralité de bandes latérales (8).

10. Casque (100) selon la revendication 9 dans la mesure où elle dépend de la revendication 3, le premier élément de connexion (6) comprenant l'au moins un élément homologue fixe (7), l'au moins un élément homologue fixe (7) étant configuré pour se coupler audit un ou auxdits plusieurs de la pluralité d'éléments fixes (18) de chacune de la pluralité de bandes latérales (8).

11. Casque (100) selon la revendication 9 ou 10 dans la mesure où elle dépend de l'une des revendications 5 à 8, la broche (5) étant configurée pour s'étendre à travers le trou traversant (3) de chacune de la pluralité de bandes latérales (8).

12. Casque (100) selon l'une des revendications 1 à 8, le premier élément de connexion (6) étant disposé sur un élément du serre-tête (2) configuré pour être placé dans une région temporale de la tête d'un utilisateur (4) ; et/ou
la première extrémité (12) de l'au moins une bande latérale (8) étant fixée par rapport au serre-tête (2).

13. Casque (100) selon l'une des revendications 1 à 12, comprenant en outre une bande médiane (20) fixée au serre-tête (2), la bande médiane (20) étant configurée pour s'étendre entre un côté avant et un côté arrière de la tête de l'utilisateur (4), l'au moins une bande latérale (8) étant fixée à la bande médiane (20).

14. Casque (100) selon l'une des revendications 1 à 11, comprenant en outre :
une première carte de circuit imprimé (24) ; et
un ou plusieurs fils (26) reliant électriquement la première carte de circuit imprimé (24) à l'au moins un contact d'électrode (14) ; et/ou
le casque (100) comprenant en outre :
une deuxième carte de circuit imprimé (32) comprenant des circuits pour collecter un signal de mesure d'électroencéphalographie provenant de la première carte de circuit imprimé (24),
la deuxième carte de circuit imprimé (32) étant couplée de manière amovible à la première carte de circuit imprimé (24).

15. Système de mesure d'électroencéphalographie (EEG) (200), comprenant :
le casque (100) selon l'une des revendications 1 à 14 ; et
au moins une électrode de mesure EEG (16),
l'au moins un contact d'électrode (14) étant connecté électriquement à l'au moins une électrode de mesure EEG (16).
